# EUROPEAN PATENT SPECIFICATION

(11) **EP 2 194 779 B1**
(45) Date of publication and mention of the grant of the patent: **21.10.2015**
(21) Application number: 08788307.0
(22) Date of filing: 12.08.2008
(51) Int. Cl.: A01N 25/02

(54) **IMPROVEMENTS IN OR RELATING TO ORGANIC COMPOUNDS**
VERBESSERUNGEN BEI ODER IN VERBINDUNG MIT ORGANISCHEN VERBINDUNGEN
AMELIORATIONS APPORTEES A DES COMPOSES ORGANIQUES OU EN RAPPORT AVEC EUX

(30) Priority: 24.08.2007 GB 0716593
(43) Date of publication of application: 16.06.2010
(73) Proprietor: Syngenta Limited, Guildford Surrey GU2 7YH (GB)
(72) Inventor: BELL, Gordon, Alastair, Berks RG42 6EY (GB); HARRIS, Clair, Louise, Berks RG42 6EY (GB); TOVEY, Ian, David, Berks RG42 6EY (GB)
(74) Representative: Syngenta International AG
(86) International application number: PCT/GB2008/002738
(87) International publication number: WO 2009/027626

(56) References cited:
- WO-A-2007/107745
- DE-A1- 4 112 873

## Description

This invention relates to compositions, particularly for agrochemical use, comprising certain lactamides and biologically active compounds, and to methods of making and using such compositions. In particular the present invention relates to such compositions when formulated as, or comprised by, an emulsion concentrate (EC).

An agrochemical (fungicidal) composition comprising dimethyl lactamide and triforine is disclosed in DE 41 12 873 A1.

Certain lactamides are disclosed in Ratchford, W. P. and Fisher, C. H., Journal of Organic Chemistry, 1950, 15, 317-325; Ratchford, W. P., Journal of Organic Chemistry, 1950, 15, 326-332; Fein, M.L. and Filachione, E.M., Journal of the American Chemical Society, 1953, 75, 2097-2099; and US 4,143,159.

Nowadays, the Formulation Chemist is required to address a number of environmental criteria when developing new formulations. Ideally, a suitable solvent will display many or all of the following properties: an excellent dissolving power for pesticides or other biologically active compounds; made from plant or animal renewable resources; low skin irritation; an ability to reduce the skin irritation associated with aggressive formulation components, such as sodium lauryl sulphate; low ecotoxicity, for example to daphnia; low volatile organic content; and a high flash point. The compositions of the present invention comprise a solvent which displays all or many of these attractive properties.

However, not all solvents are equal with respect to their capacity to dissolve biologically active compounds - the nature of the compound and its interaction with the solvent is substantially decisive. It has surprisingly been found that a particular class of solvent is surprisingly effective in dissolving a particular class of biologically active compound.

According to the present invention there is provided a composition comprising a compound of formula I

CH₃CH(OH)C(=O)NR¹R² (I)

where R¹ and R² are each independently hydrogen; or C₁₋₆ alkyl, C₂₋₆ alkenyl or C₃₋₆ cycloalkyl, each of which is optionally substituted by up to three substituents independently selected from phenyl, hydroxy, C₁₋₅ alkoxy, morpholinyl and NR³R⁴ where R³ and R⁴ are each independently C₁₋₃ alkyl; or phenyl optionally substituted by up to three substituents independently selected from C₁₋₃ alkyl; or R¹ and R² together with the nitrogen atom to which they are attached form a morpholinyl, pyrrolidinyl, piperidinyl or azepanyl ring, each of which is optionally substituted by up to three substituents independently selected from C₁₋₃ alkyl;
and at least one agrochemical selected from the group consisting of Trinexepac ethyl, Mandipropamid, Abamectin and Emamectin, with the *proviso* that the agrochemical is not abamectin or emamectin when the solvent is N-(B-hydroxyethyl)-lactamide.

The structure of Abamectin is shown in Figure 1.

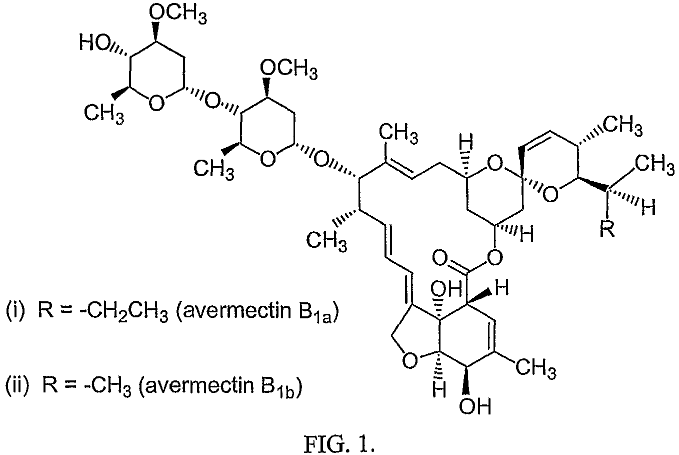

The structure of Emamectin (as the benzoate) is shown in Figure 2.

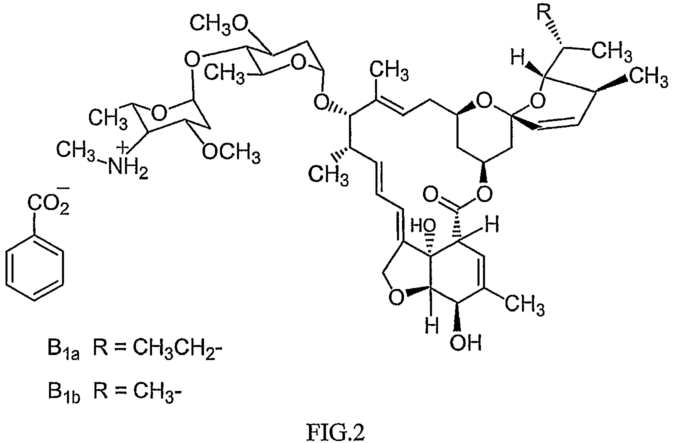

The structure of Trinexepac ethyl is shown in Figure 3.

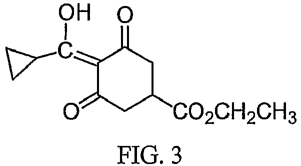

The structure of Mandipropamid is shown in Figure 4.

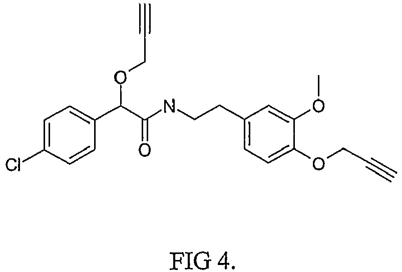

Alkyl groups and moieties are straight or branched chains. Examples are methyl, ethyl, *iso*-propyl, *n*-propyl, *n*-butyl, *sec*-butyl, *tert*-butyl, *n*-amyl and *iso*-amyl [3-methylbutyl].

Alkenyl groups and moieties may be in the form of straight or branched chains and, where appropriate, may be of either the (E)- or (Z)-configuration. Examples are vinyl and allyl.

Cycloalkyl includes cyclopropyl, cyclopentyl and cyclohexyl.

In one aspect of the composition, in the compound of formula I R¹ and R² are each independently hydrogen; or C₁₋₆ alkyl which is optionally substituted by up to three substituents independently selected from phenyl, hydroxy, C₁₋₅ alkoxy, morpholinyl and NR³R⁴ where R³ and R⁴ are each independently C₁₋₃ alkyl; or R¹ and R² together with the nitrogen atom to which they are attached form a morpholinyl ring which is optionally substituted by up to three substituents independently selected from C₁₋₃ alkyl.

In an even more suitable aspect, R¹ and R² are each independently hydrogen; or C₁₋₆ alkyl; or R¹ and R² together with the nitrogen atom to which they are attached form a morpholinyl ring.

In an even further suitable aspect, R¹ is methyl and R² is methyl, propyl or butyl; or R¹ and R² together with the nitrogen atom to which they are attached form a morpholinyl ring. R³ may be methyl, as may R⁴. For each optional substituent, it is preferred that it is a methyl group. Suitably alkyl groups are branched; most suitably with methyl groups.

In one embodiment of the composition, in the compound of formula 1, R¹ is not hydrogen, methyl, ethyl, propyl, *n*-butyl, *sec*-butyl, *iso*-butyl, *n*-amyl, *iso*-amyl, *iso-*butylenyl, *n*-hexyl, 1-3-dimethylbutyl, allyl, CH₂CH₂OH, 2-hydroxypropyl, 2-hydroxy-isobutyl, 1,3-dihydroxy-2-methyl-2-propyl, tris-hydroxy-methyl-methyl, CH₂CH₂OCH₃, cyclohexyl, phenyl, benzyl, α-methylbenzyl, β-phenylethyl, 3-hydroxypropyl or 1-hydroxy-2-butyl when R² is hydrogen;
R¹ is not methyl, allyl or phenyl when R² is methyl;
R¹ is not ethyl when R² is ethyl;
R¹ is not *n*-butyl when R² is *n*-butyl;
R¹ is not *iso*-butyl when R² is *iso*-butyl;
R¹ is not *n*-amyl when R² is *n*-amyl;
R¹ is not *iso*-amyl when R² is *iso*-amyl;
R¹ is not *n*-hexyl when R² is *n*-hexyl;
R¹ is not allyl when R² is allyl;
R¹ is not butyl or phenyl when R² is phenyl;
R¹ is not benzyl when R² is benzyl;
R¹ is not CH₂CH₂OH or ethyl when R² is CH₂CH₂OH;
R¹ is not 2-hydroxypropyl when R² is 2-hydroxypropyl; and
R¹ and R² together with the nitrogen atom to which they are attached do not form a morpholinyl, pyrrolidinyl or piperidinyl unsubstituted ring.

The composition may further comprise a solvent selected from the group consisting of aliphatic solvents; straight or branched chain paraffins; cyclic hydrocarbons; aromatic solvents; phosphorus containing solvents; sulphur containing solvents; nitrogen containing solvents; aliphatic mono, di or triesters; aromatic mono and di esters; cyclic esters; cyclic, aliphatic and aromatic ketones; alkyl cyclohexanones, dialkyl ketones, acetoacetates, benzyl ketones,; acetophenone; alcohols; cycloalcohols; glycols; glycol ethers and their polymers; propylene glycols; glycol ether acetates; aromatic alcohols; carbonates; ethers and halogenated solvents.

Particularly preferred further solvents are white oil; decalin; mono, di or tri alkylated benzenes; Solvesso 100 or 200ND (t); triethyl phosphate;tributyl phosphate; tri-2-ethylhexylphosphate;methyl oleate;linoleic acid;linolenic acid;oleic acid;dimethyl decanoamide; tetramethyl sulphone; dimethyl sulphoxide;alkyl ureas; alkanolamines; morpholines; amides; alkyl alkanoates, lactates and acetoacetates; fumarates; succinates; adipates; maleates; glycerol and citric acid esters;alkyl benzoates; benzyl alkanoates; alkyl salicylates; phthalates and dibenzoates; gamma butyrolactone; caprolactone; terpene fenchone; cyclohexanone; alkyl cyclohexanones; 2-ethylhexanol and other alkyl alcohols; cyclohexanol; tetrahydrofurfuryl alcohol; ethylene and propylene glycol and their polymers; dipropylene glycol; monomethyl or monobutyl ether; dipropylene glycol diacetate or other glycol ether acetates, or tripropylene glycol monobutyl ether; benzyl alcohol; propylene or butylene carbonate; dimethyl isosorbide; alkoxyalkanols; diphenyl ether; chlorobenzene and the chloroalkanes.

The composition may further comprise at least one compound selected from the group consisting of adjuvants, surfactants, polymers, thickening agents, dyestuffs or pigments, ultraviolet light absorbers, anti bacterial agents, salts, density modifiers, stenching or odour improving agents, taste modifiers, co solvents, and humectants. The surfactant, may be non-ionic (for example a nonylphenol ethoxylate or an alcohol ethoxylate), anionic (for example an alkyl sulphate, such as sodium lauryl sulphate, or a sulphonate, such as calcium dodecylbenzene sulphonate) or cationic (for example a tertiary amine).

The compound of formula 1 may be present in the composition in an amount of from 0.1 to 99 % by weight of the composition and the agrochemical, may be present in an amount of 0.1 to 75 %, likewise by weight.

In a preferred embodiment of the composition, the compound of formula 1 may be present in an amount of from 0.1 to 99 % by weight of the composition, the agrochemical may be present in an amount of 0.1 to 75 %, by weight and the solvent may be present in an amount of 0.1 to 90 %, likewise by weight.

The ratio of compound of formula 1 to agrochemical to solvent may be varied according to needs, a ratio of 1:1:1 or near to these limits is likely to be valuable for many of the desired formulations however the limits on each component could be as low as 0.01:1 for the ratio of any two parts of the formulation.

The lactamide component of the present compositions may be prepared by reacting a compound of formula (III) [CH₃CH(OH)C(=O)OR⁵ (III)] where OR⁵ is a leaving group, with a compound of formula (II) [HNR¹R² (II)] where R¹ and R² are as defined above. R⁵ may be C₁₋₄ alkyl. This process produces HOR⁵ as a by-product; a cleaner reaction avoids this by-product: the lactamide component of the composition of the present invention may also be prepared by reacting lactide [3,6-dimethyl-[1,4]-dioxane-2,5-dione] with a compound of formula (II) [HNR¹R² (II)] where R¹ and R² are as defined above. Schematically, such a reaction is shown below:

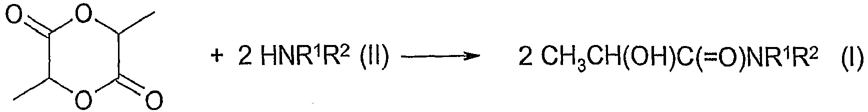

The synthesis is not limited to the above reaction scheme; it illustrates how lactide [3,6-dimethyl-[1,4]-dioxane-2,5-dione] may be converted to a lactamide by reacting lactide with an amine [suitably a primary or secondary amine] which may be carried out under "solvent-free" conditions as the skilled artisan will appreciate.

In a particularly preferred embodiment of the present inventive composition, the ratio of compound of formula 1 to agrochemical to solvent is 1:1:1 or 2:1:1 or 2:1:2 or 3:1:1 or 3:1:2 or 4.5:1:4.5 or 6:1:3 and in a still more preferred embodiment, the compound of formula 1 is dimethyl lactamide (DML).

The skilled man will recognise that the present compositions may be formulated as emulsion concentrates, emulsions in water or oil, microencapsulated formulations, aerosol sprays or fogging formulations; and these may be further formulated into granular materials or powders, for example for dry application or as water-dispersible formulations. The solutions so formed may also be used directly on soil or plants or in other, non-agrochemical, applications.

The particularly preferred formulated form of the composition is as an emulsion concentrate (EC).

The present inventive compositions have a low unwanted toxicity and excellent environmental profile meaning that they are particularly useful in applications where the minimisation of pollution is desired. Examples of such applications include paper making, water treatment, forestry applications, public health treatments, use in municipal pools and other water courses, in applications near rivers, lakes, reservoirs or seas and in applications where release to the atmosphere has to be minimised or controlled and where damage to the atmosphere is not desirable. Examples include use of fungicide containing compositions according to the present invention in exterior and interior paints, coatings, varnishes, waxes or other protectant layers or opacifiers, colourants or screens; in dyeing, pigmentation or the use of inks; in cleaning products designed for the home, garden or industrial applications; and in soap or detergent applications for industrial, home or environmental usage. The compositions of the present invention may also be used in shampoos, and in househould detergents and household cleaners (for example surface cleaners), in which the active ingredient may be a fungicide (possibly azoxystrobin) in the case of shampoo or a bacteriocide in the case of the detergents and cleaners.

The present invention also provides a method of making the inventive composition disclosed above by admixing a compound of formula 1 as indicated above with an agrochemical.

The present invention also provides the use of a composition according to any one of claims 1 to 10 in the preparation of a composition for controlling an agricultural pest by application thereof to the pest, to a locus comprising it or to a surface on which it is capable of being present.

The invention still further provides the use of the present inventive composition to control a plant pest.

The compositions of the present invention are particularly valuable in formulations where contact with either human or animal skin or eyes is required or may occur by accident. Applications such as the use of shampoo or body cleaning fluids (such as shower gels, hand or body wipes and medical wipes) may benefit from the safe nature of the lactamide solvent present in the composition, which may form part of a cleaning formulation and which may also reduce the irritancy of some of the other ingredients, such as surfactants. In a similar fashion the irritation to skin or eyes caused by the direct application of pharmaceutical or veterinary compositions to them may be reduced relative to the like application of prior art compositions containing the same pharmaceutically active ingredients.

## Claims

1. A composition comprising a compound of formula I
CH₃CH(OH)C(=O)NR¹R² (I)
where R¹ and R² are each independently hydrogen; or C₁₋₆ alkyl, C₂₋₆ alkenyl or C₃₋₆ cycloalkyl, each of which is optionally substituted by up to three substituents independently selected from phenyl, hydroxy, C₁₋₅ alkoxy, morpholinyl and NR³R⁴ where R³ and R⁴ are each independently C₁₋₃ alkyl; or phenyl optionally substituted by up to three substituents independently selected from C₁₋₃ alkyl; or R¹ and R² together with the nitrogen atom to which they are attached form a morpholinyl, pyrrolidinyl, piperidinyl or azepanyl ring, each of which is optionally substituted by up to three substituents independently selected from C₁₋₃ alkyl;
and at least one agrochemical selected from the group consisting of Trinexepac ethyl, Mandipropamid, Abamectin and Emamectin, with the *proviso* that the agrochemical is not abamectin or emamectin when the solvent is N-(B-hydroxyethyl)-lactamide.

2. A composition according to the preceding claim, wherein in the compound of formula 1 R¹ is not hydrogen, methyl, ethyl, propyl, *n*-butyl, *sec*-butyl, *iso*-butyl, *n*-amyl, *iso*-amyl, *iso*-butylenyl, *n*-hexyl, 1-3-dimethylbutyl, allyl, CH₂CH₂OH, 2-hydroxypropyl, 2- hydroxy-isobutyl, 1,3-dihydroxy-2-methyl-2-propyl, tris-hydroxy-methyl-methyl, CH₂CH₂OCH₃, cyclohexyl, phenyl, benzyl, α-methylbenzyl,
β-phenylethyl, 3-hydroxypropyl or 1-hydroxy-2-butyl when R² is hydrogen;
R¹ is not methyl, allyl or phenyl when R² is methyl;
R¹ is not ethyl when R² is ethyl;
R¹ is not *n*-butyl when R² is *n*-butyl;
R¹ is not *iso*-butyl when R² is *iso*-butyl;
R¹ is not *n*-amyl when R² is *n*-amyl;
R¹ is not *iso*-amyl when R² is *iso*-amyl;
R¹ is not *n*-hexyl when R² is *n*-hexyl;
R¹ is not allyl when R² is allyl;
R¹ is not butyl or phenyl when R² is phenyl;
R¹ is not benzyl when R² is benzyl;
R¹ is not CH₂CH₂OH or ethyl when R² is CH₂CH₂OH;
R¹ is not 2-hydroxypropyl when R² is 2-hydroxypropyl; and
R¹ and R² together with the nitrogen atom to which they are attached do not form a morpholinyl, pyrrolidinyl or piperidinyl unsubstituted ring.

3. A composition according to any preceding claim, which further comprises a solvent selected from the group consisting of aliphatic solvents; straight or branched chain paraffins; cyclic hydrocarbons; aromatic solvents; phosphorus containing solvents; sulphur containing solvents; nitrogen containing solvents; aliphatic mono, di or triesters; aromatic mono and di esters; cyclic esters; cyclic, aliphatic and aromatic ketones; alkyl cyclohexanones, dialkyl ketones, acetoacetates, benzyl ketones,; acetophenone; alcohols; cycloalcohols; glycols; glycol ethers and their polymers; propylene glycols; glycol ether acetates; aromatic alcohols; carbonates; ethers and halogenated solvents.

4. A composition according to the preceding claim, wherein the solvent is selected from the group consisting of white oil; decalin; mono, di or tri alkylated benzenes; Solvesso 100 or 200ND (t); tributyl phosphate or tris-2-ethylhexylphosphate;methyl oleate;linoleic acid;linolenic acid;oleic acid;dimethyl decanoamide; tetramethyl sulphone; dimethyl sulphoxide;alkyl ureas; alkanolamines; morpholines; amides; alkyl alkanoates, lactates and acetoacetates; fumarates; succinates; adipates; maleates; glycerol and citric acid esters;alkyl benzoates; benzyl alkanoates; alkyl salicylates; phthalates and dibenzoates; gamma butyrolactone; caprolactone; terpene fenchone; cyclohexanone; alkyl cyclohexanones; 2-ethylhexanol; cyclohexanol; tetrahydrofurfuryl alcohol; ethylene and propylene glycol and their polymers; dipropylene glycol; monomethyl or monobutyl ether; dipropylene glycol diacetate, or tripropylene glycol monobutyl ether; benzyl alcohol; propylene or butylene carbonate; dimethyl isosorbide; alkoxyalkanols; diphenyl ether; chlorobenzene and the chloroalkanes.

5. A composition according to any preceding claim, which further comprises at least one compound selected from the group consisting of adjuvants, surfactants, polymers, thickening agents, dyestuffs or pigments, ultraviolet light absorbers, anti bacterial agents, salts, density modifiers, stenching or odour improving agents, taste modifiers, cosolvents, and humectants.

6. A composition according to any preceding claim, wherein the compound of formula 1 is present in an amount of from 0.1 to 99 % by weight of the composition and the agrochemical is present in an amount of 0.1 to 75 %, likewise by weight.

7. A composition according to any one of claims 2 to 6, wherein the compound of formula 1 is present in an amount of from 0.1 to 99 % by weight of the composition, the agrochemical is present in an amount of 0.1 to 75 %, by weight and the solvent is present in an amount of 0.1 to 90 %, likewise by weight.

8. A composition according to the preceding claim, wherein the ratio of compound of formula 1 to agrochemical to solvent is defined within the limits 0.01 to 1 : 0.01 to 1 : 0.01 to 1.

9. A composition according to the preceding claim wherein the ratio of compound of formula 1 to agrochemical to solvent is 1:1:1 or 2:1:1 or 2:1:2 or 3:1:1 or 3:1:2 or 4.5:1:4.5 or 6:1:3.

10. A composition according to any preceding claim, when formulated as an emulsion concentrate (EC).

11. A method of making a composition according to any preceding claim, comprising admixing a compound of formula 1 according to claim 1 with an agrochemical according to claim 1.

12. Use of a composition according to any one of claims 1 to 10 in the preparation of a composition for controlling an agricultural pest by application thereof to the pest, to a locus comprising it or to a surface on which it is capable of being present.

13. Use of a composition according of any one of claims 1 to 10 to control a plant pest.

## Patentansprüche

1. Zusammensetzung, umfassend eine Verbindung der Formel I
CH₃CH(OH)C(=O)NR¹R² (I),
wobei R¹ und R² jeweils unabhängig voneinander für Wasserstoff; oder C₁₋₆-Alkyl, C₂₋₆-Alkenyl oder C₃₋₆-Cycloalkyl, die jeweils gegebenenfalls durch bis zu drei unabhängig voneinander aus Phenyl, Hydroxy, C₁₋₅-Alkoxy, Morpholinyl und NR³R⁴, wobei R³ und R⁴ jeweils unabhängig voneinander für C₁₋₃-Alkyl stehen, ausgewählte Substituenten substituiert sind; oder Phenyl, gegebenenfalls substituiert durch bis zu drei unabhängig voneinander aus C₁₋₃-Alkyl ausgewählte Substituenten, stehen; oder R¹ und R² zusammen mit dem Stickstoffatom, an das sie gebunden sind, einen Morpholinyl-, Pyrrolidinyl-, Piperidinyl-oder Azepanylring bilden, der jeweils gegebenenfalls durch bis zu drei unabhängig voneinander aus C₁₋₃-Alkyl ausgewählte Substituenten substituiert ist;
und mindestens eine Agrochemikalie ausgewählt aus der Gruppe bestehend aus Trinexepac-ethyl, Mandipropamid, Abamectin und Emamectin, mit der Maßgabe, dass es sich bei der Agrochemikalie nicht um Abamectin oder Emamectin handelt, wenn es sich bei dem Lösungsmittel um N-(B-Hydroxyethyl)lactamid handelt.

2. Zusammensetzung nach dem vorhergehenden Anspruch, wobei in der Verbindung der Formel I R¹ nicht für Wasserstoff, Methyl, Ethyl, Propyl, *n*-Butyl, *sek.-*Butyl, Isobutyl, *n*-Amyl, Isoamyl, Isobutylenyl, *n-*Hexyl, 1,3-Dimethylbutyl, Allyl, CH₂CH₂OH, 2-Hydroxypropyl, 2-Hydroxyisobutyl, 1,3-Dihydroxy-2-methyl-2-propyl, Trishydroxymethylmethyl, CH₂CH₂OCH₃, Cyclohexyl, Phenyl, Benzyl, α-Methylbenzyl, β-Phenylethyl, 3-Hydroxypropyl oder 1-Hydroxy-2-butyl steht, wenn R² für Wasserstoff steht;
R¹ nicht für Methyl, Allyl oder Phenyl steht, wenn R² für Methyl steht;
R¹ nicht für Ethyl steht, wenn R² für Ethyl steht; R¹ nicht für *n*-Butyl steht, wenn R² für *n*-Butyl steht;
R¹ nicht für Isobutyl steht, wenn R² für Isobutyl steht;
R¹ nicht für *n*-Amyl steht, wenn R² für *n*-Amyl steht;
R¹ nicht für Isoamyl steht, wenn R² für Isoamyl steht;
R¹ nicht für *n*-Hexyl steht, wenn R² für *n*-Hexyl steht;
R¹ nicht für Allyl steht, wenn R² für Allyl steht; R¹ nicht für Butyl oder Phenyl steht, wenn R² für Phenyl steht;
R¹ nicht für Benzyl steht, wenn R² für Benzyl steht;
R¹ nicht für CH₂CH₂OH oder Ethyl steht, wenn R² für CH₂CH₂OH steht;
R¹ nicht für 2-Hydroxypropyl steht, wenn R² für 2-Hydroxypropyl steht; und
R¹ und R² zusammen mit dem Stickstoffatom, an das sie gebunden sind, keinen unsubstituierten Morpholinyl-, Pyrrolidinyl- oder Piperidinylring bilden.

3. Zusammensetzung nach einem der vorhergehenden Ansprüche, welcher weiterhin ein Lösungsmittel ausgewählt aus der Gruppe bestehend aus aliphatischen Lösungsmitteln; geradkettigen oder verzweigten Paraffinen; cyclischen Kohlenwasserstoffen; aromatischen Lösungsmitteln; phosphorhaltigen Lösungsmitteln; schwefelhaltigen Lösungsmitteln; stickstoffhaltigen Lösungsmitteln; aliphatischen Mono-, Di- oder Triestern; aromatischen Mono- oder Diestern; cyclischen Estern; cyclischen, aliphatischen und aromatischen Ketonen; Alkylcyclohexanonen; Dialkylketonen; Acetoacetaten; Benzylketonen; Acetophenon; Alkoholen; Cycloalkoholen; Glykolen; Glykolethern und deren Polymeren; Propylenglykolen; Glykoletheracetaten; aromatischen Alkoholen; Carbonaten; Ethern und halogenierten Lösungsmitteln umfasst.

4. Zusammensetzung nach einem der vorhergehenden Ansprüche, wobei das Lösungsmittel ausgewählt ist aus der Gruppe bestehend aus Weißöl; Decalin; mono-, di- oder trialkylierten Benzolen; Solvesso 100 oder 200ND (t); Tributylphosphat oder Tris-2-ethylhexylphosphat; Ölsäuremethylester; Linolsäure; Linolensäure; Ölsäure; Dimethyldecanoamid; Tetramethylsulfon; Dimethylsulfoxid; Alkylharnstoffen; Alkanolaminen; Morpholinen; Amiden; Alkansäurealkylestern; Lactaten und Acetoacetaten; Fumaraten; Succinaten; Adipaten; Maleaten; Glycerol und Citronensäureestern; Benzoesäurealkylestern; Alkansäurebenzylestern; Salicylsäurealkylestern; Phthalaten und Dibenzoaten; gamma-Butyrolacton; Caprolacton; Terpenfenchon; Cyclohexanon; Alkylcyclohexanonen; 2-Ethylhexanol; Cyclohexanol; Tetrahydrofurfurylalkohol; Ethylen-und Propylenglykol und ihren Polymeren; Dipropylenglykolmonomethylether oder Dipropylenglykolmonobutylether; Dipropylenglykoldiacetat oder Tripropylenglykolmonobutylether; Benzylalkohol; Propylen- oder Butylencarbonat; Dimethylisosorbid; Alkoxyalkanolen; Diphenylether; Chlorbenzol und den Chloralkanen.

5. Zusammensetzung nach einem der vorhergehenden Ansprüche, welche weiterhin mindestens eine Verbindung ausgewählt aus der Gruppe bestehend aus Adjuvanzien, Tensiden, Polymeren, Verdickungsmitteln, Farbstoffen oder Pigmenten, Ultraviolettlicht absorbierenden Mitteln, antibakteriellen Mitteln, Salzen, dichtemodifizierenden Mitteln, gestank- bzw. geruchsverbessernden Mitteln, geschmacksmodifizierenden Mitteln, Kosolventien und Feuchthaltemitteln umfasst.

6. Zusammensetzung nach einem der vorhergehenden Ansprüche, wobei die Verbindung der Formel I in einer Menge von 0,1 bis 99 Gew.-% der Zusammensetzung vorliegt und die Agrochemikalie in einer Menge von 0,1 bis 75 Gew.-% vorliegt.

7. Zusammensetzung nach einem der Ansprüche 2 bis 6, wobei die Verbindung der Formel I in einer Menge von 0,1 bis 99 Gew.-% der Zusammensetzung vorliegt, die Agrochemikalie in einer Menge von 0,1 bis 75 Gew.-% vorliegt und das Lösungsmittel in einer Menge von 0,1 bis 90 Gew.-% vorliegt.

8. Zusammensetzung nach dem vorhergehenden Anspruch, wobei das Verhältnis von Verbindung der Formel I zur Agrochemikalie zum Lösungsmittel innerhalb der Grenzen von 0,01 bis 1:0,01 bis 1:0,01 bis 1 definiert ist.

9. Zusammensetzung nach dem vorhergehenden Anspruch, wobei das Verhältnis von Verbindung der Formel I zur Agrochemikalie zum Lösungsmittel 1:1:1 oder 2:1:1 oder 2:1:2 oder 3:1:1 oder 3:1:2 oder 4,5:1:4,5 oder 6:1:3 beträgt.

10. Zusammensetzung nach einem der vorhergehenden Ansprüche, die als Emulsionskonzentrat (EC) formuliert ist.

11. Verfahren zur Herstellung einer Zusammensetzung nach einem der vorhergehenden Ansprüche, bei dem man eine Verbindung der Formel I nach Anspruch 1 mit einer Agrochemikalie nach Anspruch 1 mischt.

12. Verwendung einer Zusammensetzung nach einem der Ansprüche 1 bis 10 zur Herstellung einer Zusammensetzung zur Bekämpfung eines landwirtschaftlichen Schädlings durch deren Anwendung auf den Schädling, auf einen den Schädling umfassenden Standort oder auf eine Oberfläche, auf der der Schädling vorhanden sein kann.

13. Verwendung einer Zusammensetzung nach einem der Ansprüche 1 bis 10 zur Bekämpfung eines Pflanzenschädlings.

## Revendications

1. Composition comprenant un composé de formule I
CH₃CHC(OH)C(=O)NR¹R² (I)
dans laquelle R¹ et R² sont chacun indépendamment hydrogène ; ou alkyle en C₁₋₆, alcényle en C₂₋₆ ou cycloalkyle en C₃₋₆, dont chacun est facultativement substitué par jusqu'à trois substituants indépendamment choisis parmi phényle, hydroxy, alcoxy en C₁₋₅, morpholinyle et NR³R⁴ où R³ et R⁴ sont chacun indépendamment alkyle en C₁₋₃; ou phényle facultativement substitué par jusqu'à trois substituants indépendamment choisis parmi alkyle en C₁₋₃ ; ou R¹ et R² conjointement avec l'atome d'azote auquel ils sont liés forment un cycle morpholinyle, pyrrolidinyle, pipéridinyle ou azépanyle, dont chacun est facultativement substitué par jusqu'à trois substituants indépendamment choisis parmi alkyle en C₁₋₃ ;
et au moins une substance agrochimique choisie dans le groupe constitué des trinexépac éthyl, mandipropamide, abamectine et émamectine, à condition que la substance agrochimique ne soit pas l'abamectine ou l'émamectine lorsque le solvant est le N-(B-hydroxyéthyl)-lactamide.

2. Composition selon la revendication précédente, dans laquelle, dans le composé de formule I, R¹ n'est pas hydrogène, méthyle, éthyle, propyle, *n*-butyle, *sec-*butyle, *iso*-butyle, *n*-amyle, *iso*-amyle, *iso*-butylényle, *n*-hexyle, 1-3-diméthylbutyle, allyle, CH₂CH₂OH, 2-hydroxypropyle, 2-hydroxy-isobutyle, 1,3-dihydroxy-2-méthyl-2-propyle, tris-hydroxy-méthyl-méthyle, CH₂CH₂OCH₃, cyclohexyle, phényle, benzyle, α-méthylbenzyle, β-phényléthyle, 3-hydroxypropyle ou 1-hydroxy-2-butyle lorsque R² est hydrogène ;
R¹ n'est pas méthyle, allyle ou phényle lorsque R² est méthyle ;
R¹ n'est pas éthyle lorsque R² est éthyle ;
R¹ n'est pas *n*-butyle lorsque R² est *n*-butyle ;
R¹ n'est pas *iso*-butyle lorsque R² est *is*o-butyle ;
R¹ n'est pas *n*-amyle lorsque R² est *n*-amyle ;
R¹ n'est pas *iso*-amyle lorsque R² est *iso*-amyle ;
R¹ n'est pas *n*-hexyle lorsque R² est *n*-hexyle ;
R¹ n'est pas allyle lorsque R² est allyle ;
R¹ n'est pas butyle ou phényle lorsque R² est phényle ; R¹ n'est pas benzyle lorsque R² est benzyle ;
R¹ n'est pas CH₂CH₂OH ou éthyle lorsque R² est CH₂CH₂OH ; R¹ n'est pas 2-hydroxypropyle lorsque R² est 2-hydroxypropyle ; et
R¹ et R² conjointement avec l'atome d'azote auquel ils sont liés ne forment pas un cycle morpholinyle, pyrrolidinyle ou pipéridinyle non substitué.

3. Composition selon l'une quelconque des revendications précédentes, qui comprend en outre un solvant choisi dans le groupe constitué de solvants aliphatiques ; des paraffines à chaîne linéaire ou ramifiée ; des hydrocarbures cycliques ; des solvants aromatiques ; des solvants contenant du phosphore ; des solvants contenant du soufre ; des solvants contenant de l'azote ; des mono-, di- ou triesters aliphatiques ; des mono- et diesters aromatiques ; des esters cycliques ; des cétones cycliques, aliphatiques et aromatiques ; des alkyl-cyclohexanones, des dialkylcétones, des acétoacétates, des benzylcétones ; l'acétophénone ; des alcools ; des cycloalcools ; des glycols ; des éthers de glycol et leurs polymères ; des propylène glycols ; des acétates d'éther de glycol ; des alcools aromatiques ; des carbonates ; des éthers et des solvants halogénés.

4. Composition selon la revendication précédente, dans laquelle le solvant est choisi dans le groupe constitué des huile blanche ; décaline ; benzènes mono-, di- ou trialkylés ; Solvesso 100 ou 200ND (t) ; tributylphosphate ou tris-2-éthylhexylphosphate ; oléate de méthyle ; acide linoléique ; acide linolénique ; acide oléique ; diméthyl-décanoamide ; tétraméthylsulfone ; diméthylsulfoxyde ; alkylurées ; alcanolamines ; morpholines ; amides ; alcanoates, lactates et acétoacétates d'alkyle ; fumarates ; succinates ; adipates ; maléates ; esters de glycérol et d'acide citrique ; benzoates d'alkyle ; alcanoates de benzyle ; salicylates d'alkyle ; phtalates et dibenzoates ; gamma-butyrolactone ; caprolactone ; terpéne fenchone ; cyclohexanone ; alkyl-cyclohexanones ; 2-éthylhexanol ; cyclohexanol ; alcool tétrahydrofurfurylique ; éthylène et propylène glycol et leurs polymères ; éther monométhylique de dipropylène glycol ou éther monobutylique de dipropylène glycol ; diacétate de dipropylène glycol, ou éther monobutylique de tripropylène glycol ; alcool benzylique ; carbonate de propylène ou butylène ; diméthyl-isosorbide ; alcoxyalcanols ; éther diphénylique ; chlorobenzène et chloroalcanes.

5. Composition selon l'une quelconque des revendications précédentes, qui comprend en outre au moins un composé choisi dans le groupe constitué d'adjuvants, tensioactifs, polymères, agents épaississants, colorants ou pigments, absorbeurs de lumière ultraviolette, agents antibactériens, sels, modifieurs de densité, agents contre les mauvaises odeurs ou améliorant l'odeur, modifieurs de goût, co-solvants, et humectants.

6. Composition selon l'une quelconque des revendications précédentes, dans laquelle le composé de formule I est présent en une quantité de 0,1 à 99 % en poids de la composition et la substance agrochimique est présente en une quantité de 0,1 à 75 %, également en poids.

7. Composition selon l'une quelconque des revendications 2 à 6, dans laquelle le composé de formule I est présent en une quantité de 0,1 à 99 % en poids de la composition, la substance agrochimique est présente en une quantité de 0,1 à 75 % en poids et le solvant est présent en une quantité de 0,1 à 90 %, également en poids.

8. Composition selon la revendication précédente, dans laquelle le rapport du composé de formule I à la substance agrochimique au solvant est définie dans les limites de 0,01 à 1 : 0.01 à 1 : 0.01 à 1.

9. Composition selon la revendication précédente dans laquelle le rapport du composé de formule I à la substance agrochimique au solvant est 1:1:1 ou 2:1:1 ou 2:1:2 ou 3:1:1 ou 3:1:2 ou 4.5:1:4.5 ou 6:1:3.

10. Composition selon l'une quelconque des revendications précédentes, **caractérisée en ce qu'**elle est formulée sous la forme d'un concentré en émulsion (EC).

11. Procédé de fabrication d'une composition selon l'une quelconque des revendications précédentes, comprenant le mélange d'un composé de formule I selon la revendication 1 avec une substance agrochimique selon la revendication 1.

12. Utilisation d'une composition selon l'une quelconque des revendications 1 à 10 dans la préparation d'une composition pour lutter contre un organisme nuisible agricole par application de celle-ci sur l'organisme nuisible, à un emplacement comprenant celui-ci ou sur une surface sur laquelle il est susceptible d'être présent.

13. Utilisation d'une composition selon l'une quelconque des revendications 1 à 10 pour lutter contre un organisme nuisible végétal.
